# EUROPEAN PATENT SPECIFICATION

(11) **EP 2 925 247 B1**
(45) Date of publication and mention of the grant of the patent: **09.12.2020**
(21) Application number: 13859332.2
(22) Date of filing: 21.10.2013
(51) Int. Cl.: A61B 18/04, A61N 7/02, A61H 31/00, A61H 39/06, A61M 37/00, A61F 7/00, A61F 7/12

(54) **DEVICE FOR SALVAGING MYOCARDIUM FOLLOWING HEART ATTACK**
VORRICHTUNG ZUR RETTUNG EINES MYOKARDS NACH EINEM HERZINFARKT
DISPOSITIF POUR SAUVER LE MYOCARDE À LA SUITE D'UNE CRISE CARDIAQUE

(30) Priority: 28.11.2012 US 201261730592 P; 15.10.2013 US 201314054019
(43) Date of publication of application: 07.10.2015
(73) Proprietor: Covidien LP, Mansfield, MA 02048 (US); Nau, William H. Jr., Longmont, Colorado 80504 (US)
(72) Inventor: NAU, JR., William H., Longmont, Colorado 80504 (US)
(74) Representative: Soames, Candida Jane
(86) International application number: PCT/US2013/065893
(87) International publication number: WO 2014/084984

(56) References cited:
- EP-A1- 0 957 758
- US-A- 5 538 504
- US-A- 5 775 338
- US-A- 5 961 459
- US-A- 5 968 527
- US-A1- 2002 193 785
- US-A1- 2008 140 026
- US-A1- 2010 049 284

## Description

### BACKGROUND

The present invention relates generally to systems and devices of treating a heart. More specifically, the present disclosure relates to treating damaged tissue of a heart after a heart attack using heat shock proteins.

The World Health Organization (WHO) estimates that nearly 15% of worldwide deaths are due to myocardial infarction (heart attacks) resulting from ischemic heart disease and is the leading cause of death in developing countries. Heart attacks occur when blood supply to regions of the heart is interrupted, thereby leading to ischemia and later damage or death of heart muscle tissue (myocardium). In order to improve chances for recovery after a heart attack, it may be beneficial to salvage more of the myocardium to reduce the infarct size after reperfusion. US2002193785,
EP0957758, US2008140026, US5538504 and US5961459 refer to prior art relevant for the present invention, the latter is considered to be the closest prior art.

### SUMMARY

The described features generally relate to one or more improved systems, and/or apparatuses for treating the heart, such as treating ischemia in heart tissue after a heart attack. The heart tissue may be treated by providing heat shock proteins (HSPs) in the tissue. The heat shock proteins may provide myocardial protection for the heart tissue. The heat shock proteins are provided using, an energy-based device that generates heat in the heart tissue, which stimulates automatic generation of the heat shock proteins by the tissue. The energy-based device includes an ultrasound device. Additionally, the heat shock proteins are applied onto a surface of or injected into the heart tissue. The energy-based device is used to drive the heat shock proteins into the heart tissue. The invention is defined by appended claim 1.

An example method may be directed to a method of treating ischemia in heart tissue after a heart attack, The method can include providing a heat treatment device comprising a heating element, positioning the heating element in proximity to the heart tissue, and heating the heart tissue with the heating element to generate release of heat shock proteins in the heart tissue. The heat shock proteins can provide myocardial protection for the heart tissue.

Heating the heart tissue can include directing ultrasound waves into the heart tissue. The ultrasound waves can have a frequency in the range of about 1 MHz to about 10 MHz. The method can include delivering a drug to the heart tissue, wherein the drug comprises heat shock proteins. The method can include driving the drug into the heart tissue with the ultrasound waves. The method can include injecting the drug into the heat tissue. The drug can comprise an adenovirus vector.

Another example method may be directed to method of providing myocardial protection in the heart after myocardial infarction. The method can include providing a heart tissue treatment device, positioning the heart tissue treatment device in proximity to the heart, and utilizing the heart tissue treatment device to provide heat shock proteins to tissue of the heart for myocardial protection.

Positioning the heart tissue treatment device can include positioning a portion of the heart tissue treatment device in contact with an exterior surface of the heart. The method can include heating the tissue using the heart tissue treatment device to generate the heat shock proteins. Positioning the heart tissue treatment device can include inserting a portion of the heart tissue treatment device through a vessel into the heart. The method can include providing a fluid interface between the heart tissue treatment device and the tissue of the heart.

A further example method may be directed to a method of treating heart tissue damaged during a heart attack. The method can include reperfusing an artery of the heart to create blood flow to the heart tissue, and heating the heart tissue to a temperature of no more than about 7°C above body temperature to trigger release of heat shock proteins in the heart tissue.

Heating the heart tissue includes generating heat using an ultrasound device. Heating the heart tissue can include heating from outside of the heart. Alternatively, heating the heart tissue can include heating from inside of the heart. The method can include heating the heart tissue to a temperature of about 37°C to about 44°C. Heating can be provided by a heating element, and a fluid interface can be provided between the heating element and the heart tissue. The method can include providing a heating element carried by a catheter, which is inserted through a vessel to the heart. The catheter can include a reperfusion member operable to provide the reperfusing, and the heating element can be positioned on the catheter at a location distal of the reperfusion member.

A heart treatment device can include a delivery member, a heating element, and a tissue interface portion. The delivery member can have a distal support portion. The heating element can be carried by the distal support portion and configured to generate heat to increase a temperature in heart tissue. The tissue interface portion can be positioned between the heating element and the heart tissue.

The heating element includes an ultrasound device. The ultrasound device can generate ultrasound in the range of about 1 MHz to about 10 MHz. The delivery member can include an elongate instrument configured to be inserted through a wall of a patient's chest cavity to access the heart. The heating element can be positioned outside of the heart. The tissue interface can include a fluid-filled balloon. The delivery member can include a catheter that is insertable within the heart. The catheter can comprise a reperfusion member positioned proximal of the heating element. The reperfusion member can comprise at least one of an expandable balloon and a stent. The tissue interface can comprise a vest carried by a patient. The vest can position the heating element along an exterior of the patient at a predetermined location relative to the heart. The heating element can comprise a rectangularly shaped radially outward facing surface.

The foregoing has outlined rather broadly the features and technical advantages of examples according to the disclosure in order that the detailed description that follows may be better understood. Additional features and advantages will be described hereinafter. The conception and specific examples disclosed may be readily utilized as a basis for modifying or designing other structures for carrying out the same purposes of the present disclosure. Such equivalent constructions do not depart from the scope of the appended claims. Features which are believed to be characteristic of the concepts disclosed herein, both as to their organization and method of operation, together with associated advantages will be better understood from the following description when considered in connection with the accompanying figures. Each of the figures is provided for the purpose of illustration and description only, and not as a definition of the limits of the claims.

### BRIEF DESCRIPTION OF THE DRAWINGS

A further understanding of the nature and advantages of the embodiments may be realized by reference to the following drawings. In the appended figures, similar components or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.
FIG. 1 shows schematically a heart treatment device used to treat damaged tissue in a heart.
FIG. 2 shows an example heart treatment device contacting an outer surface of a heart in accordance with the present disclosure.
FIG. 3 shows the heart treatment device of FIG. 2 advanced through a chest wall of a patient and into contact with an outer surface of the heart.
FIG. 4 shows a distal portion of another example heart treatment device in accordance with the present disclosure.
FIG. 5 shows a distal end portion of another example heart treatment device in accordance with the present disclosure.
FIG. 6 shows a distal end portion of another example heart treatment device in the form of a catheter in accordance with the present disclosure.
FIG. 7 shows a distal end portion of another example heart treatment device in the form of a catheter in accordance with the present disclosure.
FIG. 8 shows the heart treatment of FIG. 7 inserted through a vessel of a heart,
FIG. 9 shows a distal end portion of another example heart treatment device inserted in a heart vessel in accordance with the present disclosure.
FIGS. 10A and 10B show a distal end portion of another example heart treatment device in combination with a reperfusion device in accordance with the present disclosure.
FIG. 11 shows another example heart treatment device for use in treating a heart from outside of a patient's body.
FIG. 12 shows a side view of the heart treatment device of FIG. 11 treating a heart.
FIG. 13 shows another example heart treatment device for treating a heart from outside the patient's body.
FIG. 14 shows a side view of the heart treatment device of FIG. 13 treating a heart.
FIGS. 15-17 are flow diagrams showing example methods of treating a heart in accordance with the present disclosure.

### DETAILED DESCRIPTION

The present invention generally relates to systems and devices for treating a heart after a heart attack. Specifically, the systems, devices and methods aid in the protection of the myocardium following a heart attack. One aspect of the present disclosure relates to using heat shock proteins (HSP) to treat heart tissue. The heat shock proteins are generated by the heart tissue itself upon heating the heart tissue. Additionally, the heat shock proteins are delivered to the heart tissue via drug delivery.

Generally, heat shock proteins are a group of proteins that are produced when cells are exposed to sub-lethal temperature elevations. Heat shock proteins act to protect cells from damage due to further induced stresses. One produced, these proteins may provide similar protection from non-thermal stresses as well. A specific heat shock protein, HSP70, has been shown to play a key role in myocardial protection following ischemia. The elevation of HSP70 within 24 hours after ischemia may have particular advantages related to myocardial salvage.

Heat shock proteins have been shown to provide myocardial protection following ischemia and myocardial infarction. One way to generate heat in the heart tissue to trigger release of the heat shock proteins involves an ultrasound-based device capable of heating the myocardium. The ultrasound-based device is positioned in proximity to the heart and generates ultrasonic waves that pass into the heart issue. The ultrasound-based device may be positioned in contact with an exterior surface of the heart and the ultrasound waves pass through the exterior surface into the heart tissue. In another example, the ultrasound-based device is advanced through an artery of the heart and the ultrasound waves pass through the artery wall into the heart tissue. In another example, the ultrasound-based device is advanced into a chamber of the heart and the ultrasound waves pass through the chamber wall into the heart tissue. In a still further example, the ultrasound-based device is positioned spaced apart from the heart tissue and is separated by, for example, the patient's chest wall (*e.g.,* a rib cage of the patient). The ultrasound-based device may be arranged in contact with an exterior surface of the chest wall.

According to the invention, the heart treatment device uses ultrasound as the heating source. The ultrasound generating device is not only generating heat in the heart tissue, but is also aiding in the delivery and rapid diffusion of the drug due to the streaming effect of the ultrasound.

In one embodiment, the devices and methods disclosed herein are intended to elevate temperatures in the infarct region of the heart to a temperature in the range of about 37°C to about 45°C, and more preferably in the range of about 38°C to about 42°C. In some examples, the temperature elevation is no more than about 4°C above body temperature. This temperature range may stimulate the synthesis of heat shock proteins, such as HSP70, thereby protecting the myocardium after reperfusion. This range may also avoid problems of damaging the heart tissue, which may occur at higher temperatures.

Reperfusion of an arterial vessel, the blockage of which results in the damaged heart tissue, may be completed using, for example, stenting or balloon angioplasty. As mentioned above, the heart treatment device may be used to deliver an HSP70 promoting gene via an adenoviral vector, The heart treatment device may also increase diffusion of the drug through the tissue using the streaming effect of ultrasound or other energy source, While an ultrasound-based device is emphasized throughout the present disclosure, other types of heating devices may be used to stimulate the synthesis of HSP70 and other heat shock proteins. For example, the heart treatment device may generate heat in the heart tissue using one of a radio frequency device, a resistive heating device, a laser heating device, a microwave heating device, or a flow of hot fluid such as water.

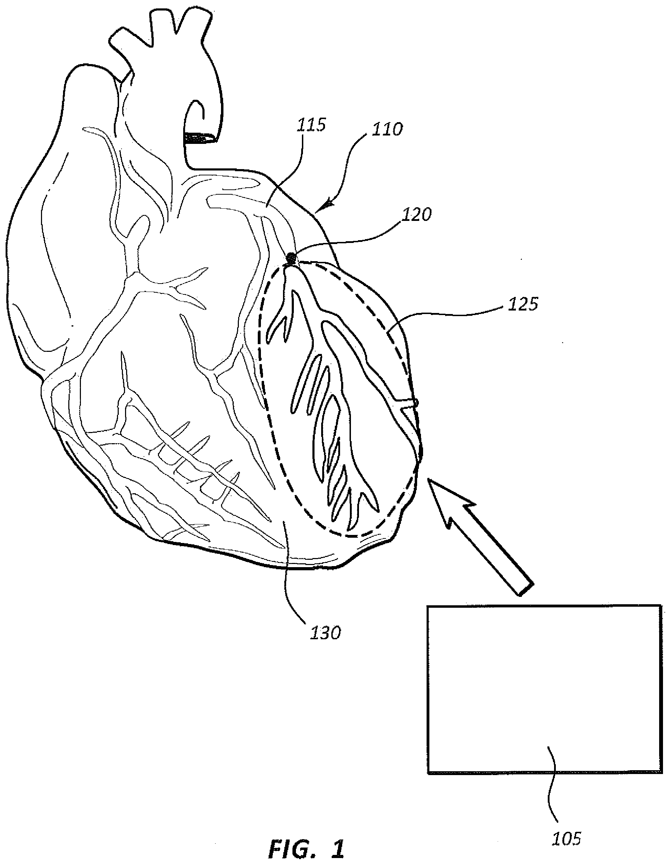

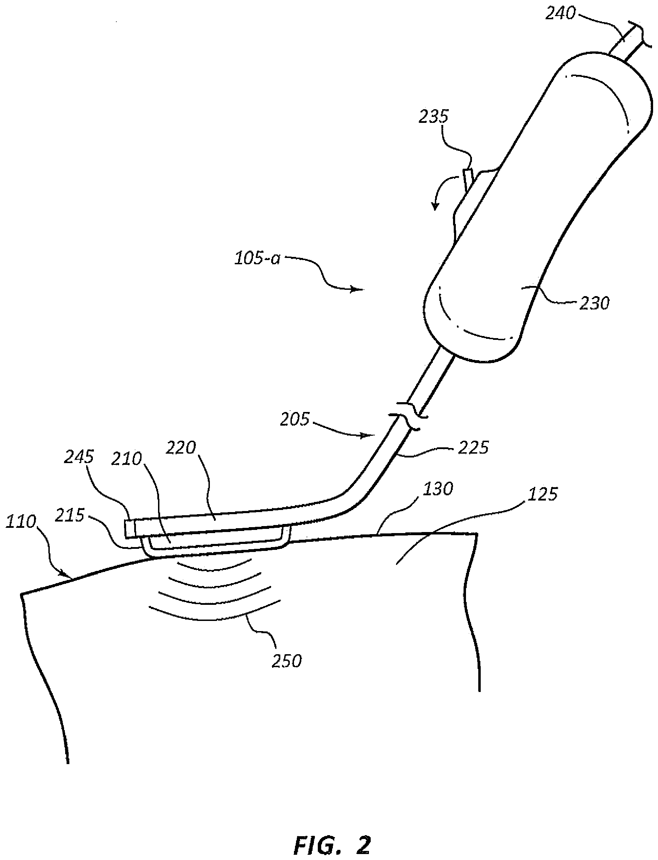

Referring now to FIG. 1, an example heart treatment device 105 is shown in proximity to a heart 110. The heart treatment device 105 is used to treat damaged heart tissue 125 resulting from a blockage 120 in a coronary artery 115. The heart treatment device 105 may use one of a variety of treatment methods to provide heat shock proteins in the heart tissue 125. The heat shock proteins are generated using an injection of drugs as will be described in further detail below. Additionally, the heat shock proteins are generated within the heart tissue by heating the heart tissue 125. Several example heart treatment devices are described in further detail with reference to FIGS. 2-14.

FIG. 2 shows a heart treatment device 105-a, which may be an example of the heart treatment device 105 of FIG. 1. The heart treatment device 105-a includes a delivery member 205, a heating element 210, and a tissue interface portion 215. The heart treatment device 105-a may be operable to treat the heart tissue 125 from outside of the heart 110. In one example, the heart treatment device 105-a is positioned with the tissue interface portion 215 in contact with an outer surface 130 of the heart 110. Operating the heating element 210 may generate heat within the heart tissue 125, which stimulates the synthesis of heat shock proteins, such as HSP70.

The delivery member 205 includes a distal support portion 220, a support arm 225, and a handle 230. The distal support portion 220 is configured to position the heating element 210 and tissue interface portion 215 adjacent to the outer surface 130 of the heart 110. The handle 230 may be configured for easy grasping by an operator. The support arm 225 extends from the handle 230 and supports the distal support portion 220 at a distal end thereof.

The delivery member 205 may further include a controller 235 used to operate the heating element 210. The controller 235 may be mounted to the handle 230. The controller 235 may be operable between on and off positions. The controller 235 may be configured as a thumb-actuated device accessible along an exterior of the handle 230. In other arrangements, the controller 235 is positioned remote from the handle 230 and controlled in other ways using, for example, a foot-actuated pedal or an on/off switch at a home unit to which the delivery member 205 is connected via, for example, a cable that extends collinearly with the power source 240.

The heating element 210, when powered, may generate an output 250 (e.g., in the form of a pressure wave or energy wave) that passes through the tissue interface portion 215 into the heart tissue 125. In one example, the heating element 210 is an ultrasound device that generates ultrasound waves, which are absorbed by the heart tissue 125 to heat up the heart tissue 125. The ultrasound waves device may generate a continuous wave mode that can be turned on and off according to a desired sequence to generate and maintain a desired temperature in the heart tissue 125.

The heating element 210 may have various sizes and shapes. In one example, the heating element 210 has a generally rectangular-shaped, planar face directed toward the heart tissue 125. The face of the heating element 210 may be modified so that energy comes out of only a portion of the face to provide improved directionality of the energy output. In one example, the output face of the heating element 210 has a convex curvature, which may broaden the direction in which the energy is directed away from the heating element 210. In another example, the heating element 210 has a concave curvature, which may narrow the direction in which energy is directed. Another example heating element may have a cylindrical shape with a generally circular cross-section. The cylindrical shape of the heating element 210 may provide the curved shape described above.

The tissue interface portion 215 provides an interface between the heating element 210 and the heart tissue 125. The tissue interface portion 215 may comprise a compliant structure that provides improved contact with the outer surface 130 of the heart 110. The tissue interface portion 215 may comprise a balloon structure that is filled with fluid. The fluid may comprise, for example, a de-gassed saline, a de-gassed, de-ionized water, glycerin or oil. The tissue interface portion 215 may be inflated with the fluid prior to or after contacting the outer surface 130 of the heart 110. In at least some examples, the tissue interface portion 215 is deflated during insertion of the heart treatment device 105-a into the patient to help minimize an outer profile of the heart treatment device 105-a, and later inflated it to provide an improved (e.g., enlarged) interface between the heating element 210 and the heart 110.

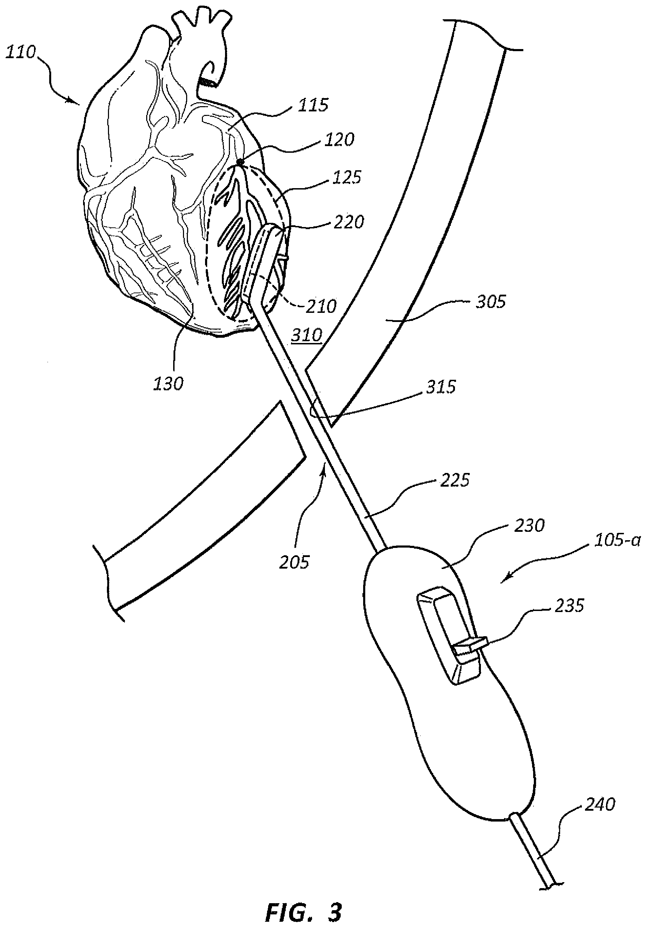

FIG. 3 shows the heart treatment device 105-a of FIG. 2 inserted through incision 315 in a chest wall 305 to gain access to a chest cavity 310 adjacent to the heart 110. The heating element 210 may be positioned adjacent to the outer surface 130 of the heart 110 with the tissue interface portion 215 interposed therebetween and in contact with the outer surface 130. The incision 315 may be a puncture formed, for example, just under the ribcage or through an intercostal space. In some examples, a trocar or a sleeve may be prepositioned extending through the chest wall 305 to provide a path for delivery of the heart treatment device 105-a through the chest wall 305.

Accessing the heart 110 through the chest wall 305 may provide certain advantages as compared to delivery of the heart treatment device 105-a through other paths such as, for example, through an artery, or by performing open heart surgery in which the ribcage is broken. One advantage relates to being able to treat the heart 110 with the heart treatment device 105-a while other procedures are conducted on the heart including, for example, a percutaneous catheter intervention to provide reperfusion through the coronary artery 115 that includes blockage 120. The heart treatment device 105-a may be used to treat the heart tissue 125 from outside of the heart 110 while a percutaneous catheter intervention is conducted in an intra-cardiac procedure. Treatment using the heart treatment device 105-a may occur prior to, during, and after the percutaneous catheter intervention procedure.

The heart treatment device 105-a may be used with an ultrasound imaging device that is also inserted into the chest cavity 310. The ultrasound imaging device or other suitable imaging device may be used to view the heart 110 to help position the heating element 210 in proximity to the outer surface 130 of heart 110. The imaging device may be mounted directly to the heart treatment device 105-a. Alternatively, the imaging device may be carried by a separate device that is inserted through a separate incision in the chest wall 305. The imaging device may include a light source to illuminate objects within the chest cavity 310.

Referring again to FIG. 2, the heart treatment device 105-a may include a temperature sensor 245 carried by, for example, the distal support portion 220. The temperature sensor 245 may monitor a temperature of the heart tissue 125 during operation of the heart treatment device 105-a. In at least some examples, the desired temperature for the heart tissue 125 is in the range of about 37°C to about 44°C, and more preferably in the range of about 38°C to about 42°C. The temperature of heart tissue 125 may typically be raised no more than about 7°C above body temperature (e.g., normal body temperature being about 37°C).

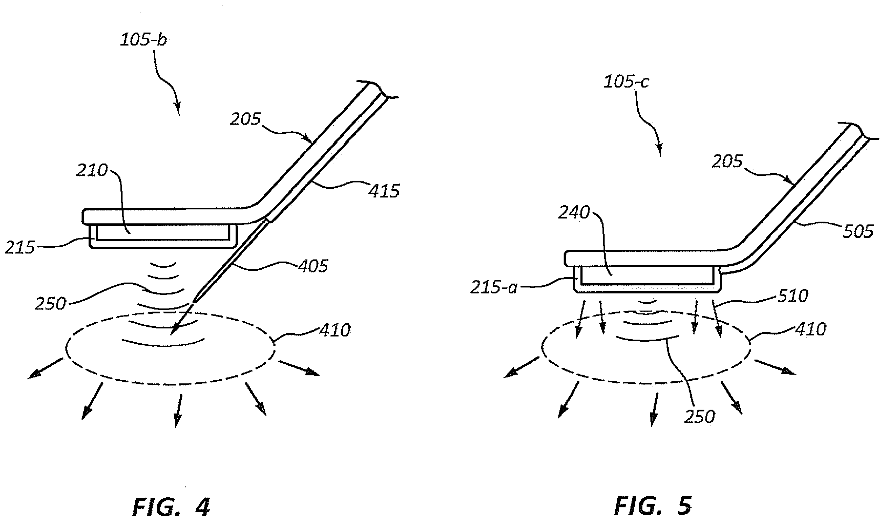

In embodiments where an ultrasound device is used for the heating element 210, the ultrasound device may be operated at a relatively low frequency such as, for example, in the range of about 1 MHz to about 10 MHz, and more preferably in the range of about 3 MHz to about 8 MHz. As mentioned above, the ultrasound device may be operated in a continuous wave mode in which the heart tissue 125 absorbs the ultrasound energy and heats up. The ultrasound wave generated by the ultrasound device may create a pushing force that may be used to advance fluids within the heart tissue 125 in a direction away from the heating element 210. This pushing force may be helpful when attempting to dissipate fluids such as a drug that are injected into the heart tissue 125, as will be described below with reference to FIGS. 4-5 and 8-9.

The heating element 210 may be operated for different amounts of time depending on, for example, the temperature attained in the heart tissue 125, the frequency of the output (e.g., measured in MHz for an ultrasound device), and the shape and size of the heating element 210. In one example, the heart tissue 125 is maintained at a raised temperature for a time period of less than about an hour, and more preferably in a range of about 10 minutes to about 30 minutes. Multiple treatments are possible in which the temperature of the heart tissue 125 is increased and maintained for a certain period of time, followed by cooling of the heart tissue 125, and then reheating the heart tissue 125. For example, it may be possible to provide three treatment phases that include heated periods of, for example, 20 minutes to 30 minutes, and intermediate stay periods of about 5 minutes to about 15 minutes.

FIG. 4 shows a heart treatment device 105-b, which may be another example of the heart treatment device 105 of FIG. 1. The heart treatment device 105-b includes a delivery member 205, a heating element 210, a tissue interface portion 215, and a needle 405. The needle 405 may be used to deliver a drug 410 directly into the heart tissue 125. The drug 410 may be delivered prior to, during or after operating the heating element 210. In some examples, the drug 410 is delivered through needle 405 followed by operating the heating element 210 to heat the heart tissue 125. The heating element 210 includes an ultrasound device that creates a pressure wave 250 that drives and dissipates the drug 410 within the heart tissue 125.

The drug 410 comprises heat shock protein promoting gene via an adenoviral vector. The heat shock protein may be, for example, HSP70.

The needle 405 may be operated to advance into the heart tissue 125 to deliver the drug 410, and later be withdrawn into a needle lumen 415. Advancing and retracting the needle 405 may be controlled at the handle of the delivery device (e.g., the handle 230 shown with reference to FIG. 2).

FIG. 5 shows a heart treatment device 105-c, which may be a further example of the heart treatment device 105 of FIG. 1. The heart treatment device 105-c includes a delivery member 205, a heating element 210, and a tissue interface portion 215-a. The tissue interface portion 215-a may act as a drug delivery interface for delivering a drug 410 into the heart tissue 125. The drug may be delivered through a drug delivery lumen 505. The tissue interface portion 215-a may comprise a fluid permeable material that permits the drug to leak through the tissue interface portion 215-a into the heart tissue 125.

The tissue interface portion 215-a may comprise a plurality of holes of a size that permits passage of the drug 410 when a fluid pressure threshold is met within the tissue interface portion 215-a. The drug 410 may flow in a fluid path 510 away from the heating element 210 into the heart tissue 125. Operating the heating element 210 may drive the drug 410 for further dissipation within the heart tissue 125. In at least one example, the heating element 210 comprises an ultrasound device that creates a pressure wave that assists in dissipating the drug 410 within the heart tissue 125.

The drug delivery lumen 505 may extend along the delivery member 205 to the handle (e.g., similar to handle 230 of heart treatment device 105-a), wherein a source of the drug is controlled for delivery to the tissue interface portion 215-a.

The drug 410 may be delivered using alternative features and methods. For example, the drug 410 may be deposited on the outer surface 130 of the heart 110 directly from the drug delivery lumen 505 and without the tissue interface portion 215-a or the needle 405. Furthermore, the multiple drug delivery features may be included on the same heart treatment device. For example, the needle 405 and tissue interface portion 215-a may be included on the same heart treatment device for delivery of a drug to the heart 110.

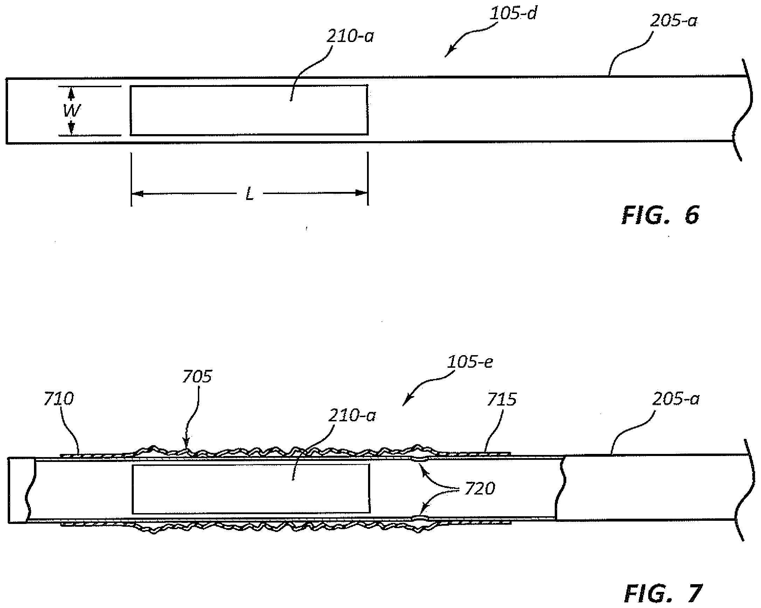

FIG. 6 shows a heart treatment device 105-d, which may be an example of the heart treatment device 105 of FIG. 1. The heart treatment device 105-d is in the form of, for example, a flexible, semi-flexible, or rigid catheter configured for insertion through a vessel or other lumen into the heart. The heart treatment device 105-d includes a delivery member 205-a and a heating element 210-a. The heating element 210-a may be an example of the heating element 210 described above, and may be uniquely designed for use with a catheter. The delivery member 205-a may be constructed as a catheter that is insertable through a vessel such as a femoral artery, and into the heart. The heating element 210-a may have a length L and a width W that fits within an outer profile of the delivery member 205-a. In one example, the heating element 210-a has a plate-shape with a generally planer surface that directs energy in a specific direction. In other examples, the heating element 210-a emanates energy in multiple directions. In one embodiment, the heating element 210-a has a cylindrical shape. The heating element 210-a may be embedded in the delivery member 205-a. A portion of the delivery member 205-a may act as a tissue interface portion that provides an interface with an internal surface of the vessel.

The heart treatment device 105-d may be positioned within any cavity of the heart including, for example, one of the chambers of the heart, a coronary artery, or one of the arteries or veins leading into or out of the heart.

FIG. 7 shows a heart treatment device 105-e, which may be an example of the heart treatment device 105 of FIG. 1 and may include some of the features of heart treatment device 105-d. The heart treatment device 105-e includes a delivery member 205-b, a heating element 210-a and a tissue interface portion 705. The tissue interface portion 705 may include distal and proximal waists 710, 715 used to secure the tissue interface portion 705 to the delivery member 205-b. The tissue interface portion 705 may be constructed as a balloon having a fluid path 720 for delivery of inflation fluid into the tissue interface portion 705. The tissue interface portion 705 may be expandable.

The tissue interface portion 705 may be deflated to minimize an outer profile of the heart treatment device 105-e for delivery of the heart treatment device 105-e to the heart 110. The tissue interface portion 705 may be later inflated (e.g., via the fluid path 720) to provide improved contact between the heart treatment device 105-e and surfaces of the cavity within which the heating element 210-a is positioned.

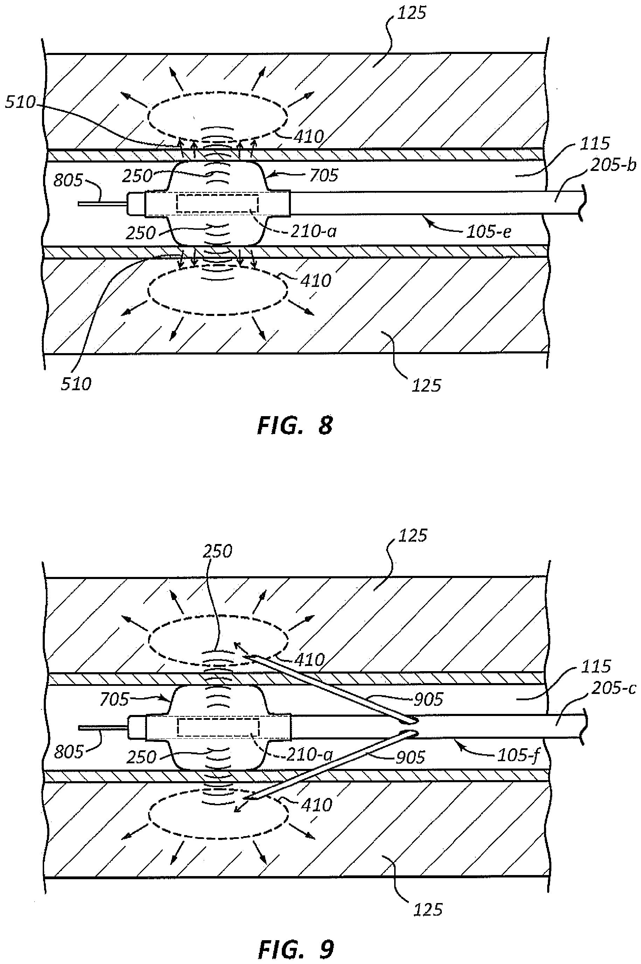

FIG. 8 shows the heart treatment device 105-e positioned within a coronary artery 115 adjacent to damaged heart tissue 125. The tissue interface portion 705 is inflated to provide contact between the heart treatment device 105-e and an internal surface of the coronary artery 115. The heating element 210-a is operated to heat the heart tissue 125. In at least some examples, the tissue interface portion 705 may be used to deliver a drug 410 to the heart tissue 125. The drug may pass through the tissue interface portion 705, through a wall of the coronary artery 115 and into the heart tissue 125. The heating element 210-a may be used to dissipate the drug 410 within the heart tissue 125 (e.g., via a pressure wave generated by an ultrasound device). Alternatively, the drug 410 may be delivered independent of operating the heating element 210-a. In some embodiments, the heart treatment device 105-e may be operable to provide a heat shock protein to the heart tissue 125 without using a heating element.

The heart treatment device 105-e may be advanced over a guide wire 805 to position the heart treatment device 105-e at a desired location within the coronary artery 115. The guide wire 805 may be prepositioned within the coronary artery 115 or within some other cavity of the heart 110.

FIG. 9 shows a heart treatment device 105-f, which may be an example of the heart treatment device 105 of FIG. 1 and may include some of the features of heart treatment device 105-d. The heart treatment device 105-f includes a delivery member 205-c, a heating element 210-a, a tissue interface portion 705, and a pair of needles 905. The needles 905 may be used to deliver a drug 410 to the heart tissue 125. The needles 905 may be advanced from the delivery member 205-c, through the wall of the coronary artery 115, and directly into the heart tissue 125. After delivering the drug 410, the needles 905 may be withdrawn back into the delivery member 205-c. In other embodiments, the heart treatment device 105-f includes separate needle lumens positioned outside of or carried by the delivery member 205-c within which the needles 905 are carried.

The heating element 210-a may operate to heat the heart tissue 125. The heating element 210-a may generate pressure waves 250 or other forces that help dissipate the drug 410 within the heart tissue 125. Heating the heart tissue 125 may generate heat shock proteins such as HSP70. Likewise, the drug 410 may include HSP70 or other heat shock proteins, or a heat shock protein promoting gene delivered via, for example, an adenoviral vector.

The heart treatment device 105-f may include a number of needles 905. The needles 905 may be directed in any desired direction including, for example, in a lateral, curved, or proximal direction as opposed to the generally distal direction shown in FIG. 9. In some embodiments, the needles 905 may protrude directly radially outward from the delivery member 205-c. Further, the needles 905 may protrude from the delivery member 205-c at a location distal of the heating element 210-a as opposed to the proximal orientation shown in FIG. 9.

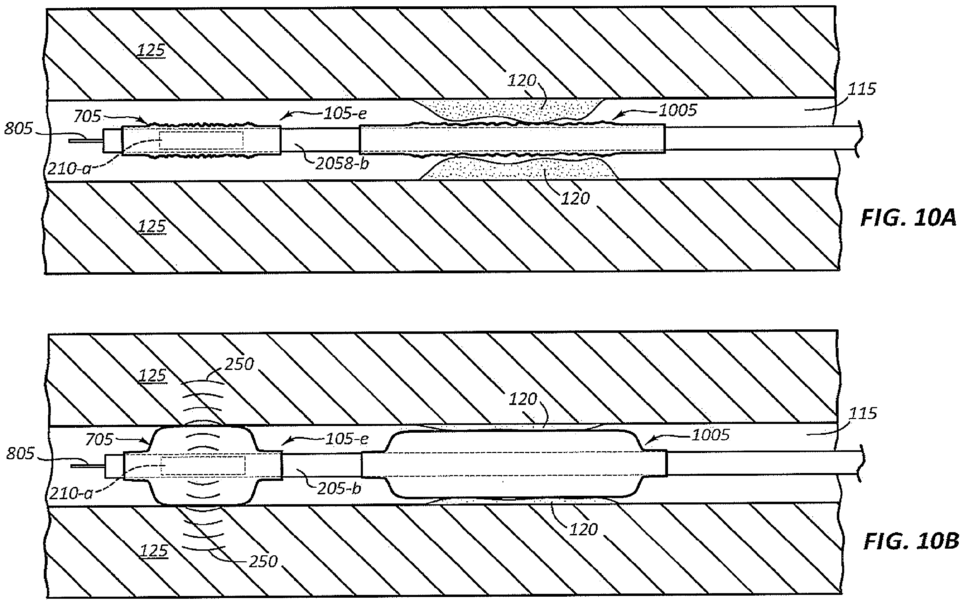

The heart treatment device 105-e shown and described with reference to FIGS. 7 and 8 may be combined on the same catheter with a reperfusion member 1005 as shown in FIGS. 10A and 10B. The reperfusion member 1005 may be positioned at a location on the delivery member 205-b proximal of the heating element 210-a. The reperfusion member may be positioned spanning the blockage 120 in coronary artery 115 (*see* FIG. 10A), and operated to compress the blockage 120 as shown in FIG. 10B. The reperfusion member 1005 may comprise, for example, a stent or inflation balloon.

The heating element 210-a and tissue interface portion 705 may be positioned distal of the reperfusion member 1005. Typically, the damaged heart tissue 125 is positioned distal of the blockage 120. The heating element 210-a may be positioned radially adjacent to the damaged heart tissue 125 and used to treat the heart tissue 125 prior to, during, or after operation of the reperfusion member 1005,

In other arrangements, the heating element 210-a and needles 905 are positioned proximal of the reperfusion member 1005. The reperfusion member 1005 may operate to treat the blockage, followed by further advancing the heart treatment device past the blockage to position the heating element 210-a and needles 905 adjacent to the heart tissue 125 to treat the heart tissue 125.

In at least some scenarios, reperfusion of the myocardial cells previously starved of blood flow may lead to additional loss of cells (*i.e.,* reperfusion injury). The release of heat shock proteins in the heart tissue 125 may also aid in protection against the negative effects of the reperfusion, particularly if the heat shock proteins are provided in advance of restoring flow.

Providing the heart treatment device 105-e in combination with the reperfusion member 1005 may also limit the amount of time needed to treat the heart tissue 125 by providing heat shock proteins. In other embodiments in which the reperfusion member 1005 must be inserted, operated and withdrawn prior to advancing the heart treatment device 105-c through the vessel to the heart tissue 125, the combined device shown in FIGS. 10A and 10B may significantly reduce the amount of time delay between reperfusion and treatment of the heart tissue 125.

There are various ways in which to provide the reperfusion member and heart treatment device within a coronary artery 115 at the same time. For example, separate catheters may be inserted side by side through a vessel, wherein one of the catheters is the delivery member 205-b and the other catheter carries the reperfusion member 1005. Alternatively, the separate catheters may be inserted through separate vessels, which are positioned adjacent to each other in the heart.

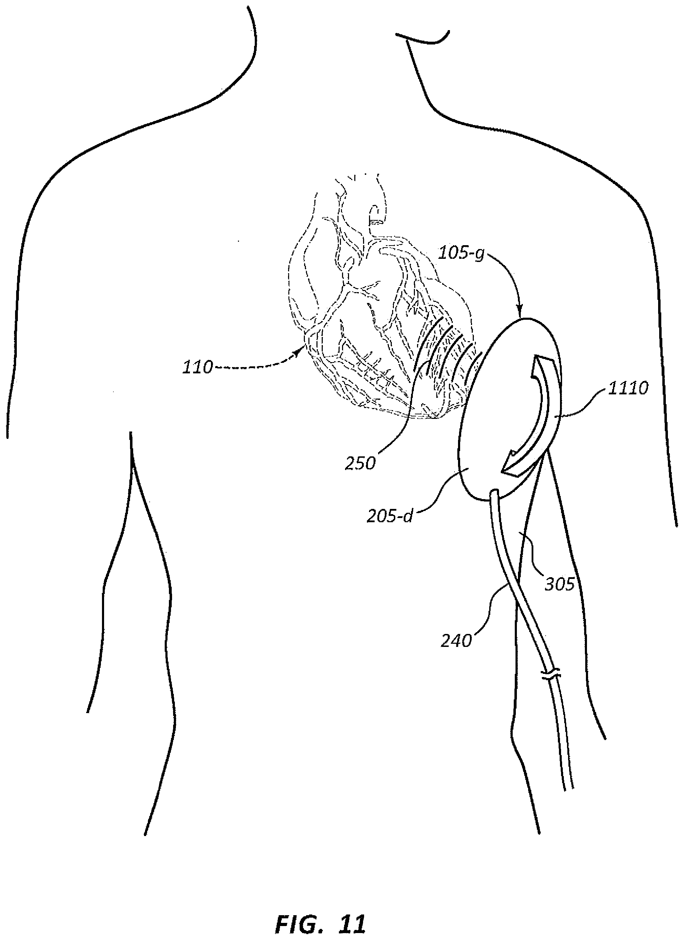

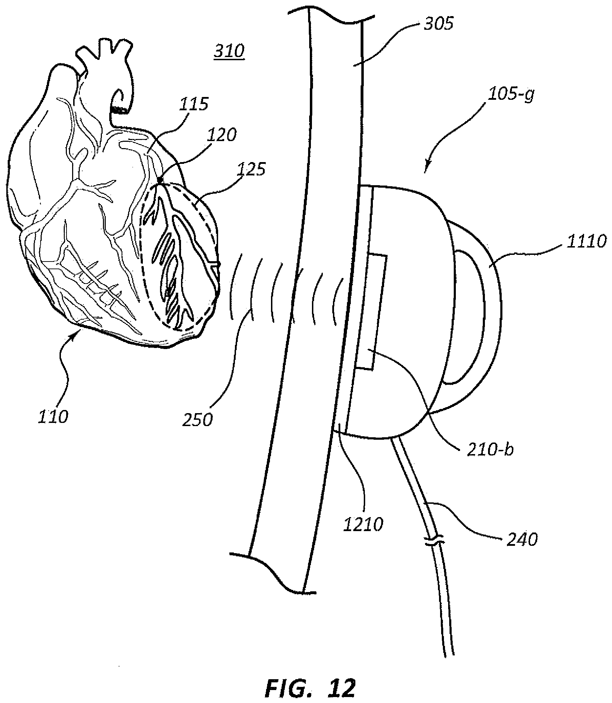

FIGS. 11 and 12 show a heart treatment device 105-g, which may be an example of the heart treatment device 105 of FIG. 1. The heart treatment device 105-g includes a delivery member 205-d, a heating element 210-b, a tissue interface portion 1210 and a power source 240. The delivery member 205-d may include a handle 1110 used to position the heart treatment device 105-g along an exterior surface of the chest wall 305. The heating element 210-b may operate to direct energy to damaged heart tissue 125 of the heart 110. The energy directed from the heating element 210-b may pass through the chest wall 305 and through the chest cavity 310 to the heart 110. The operator may move the heart treatment device 105-g along the outer surface of the chest wall 305 to direct the energy waves 250 to a desired portion of the heart 110, which preferably includes the damaged heart tissue 125.

The heart treatment device 105-g may be used in combination with other devices such as, for example, an x-ray machine or other suitable imaging machines that show the position of the heart 110 relative to the heart treatment device 105-g. The heart treatment device 105-g may operate completely non-invasively. The heart treatment device 105-g may operate without penetrating or disturbing any layers or tissue of the patient.

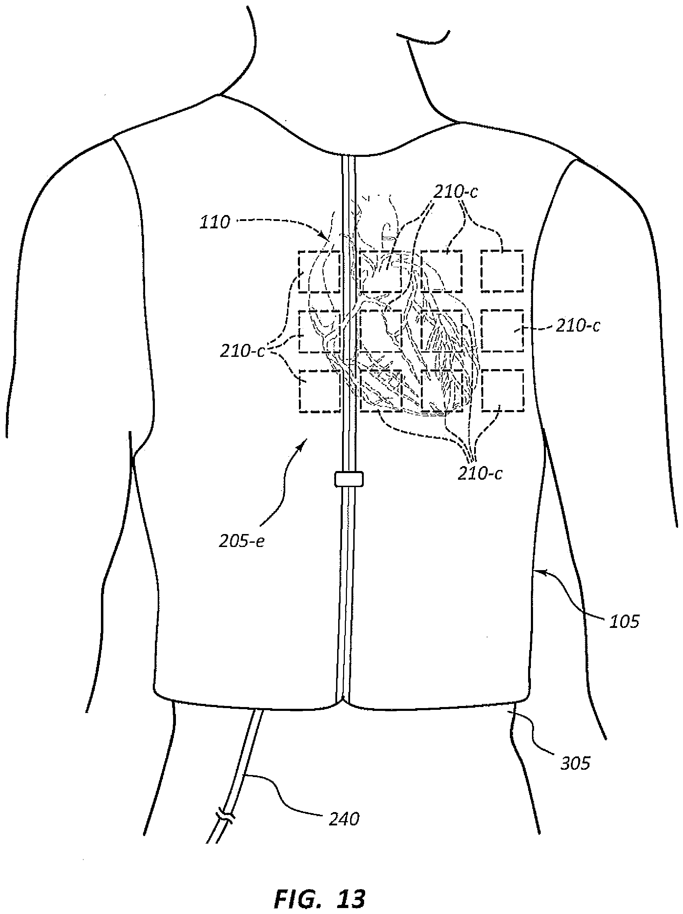

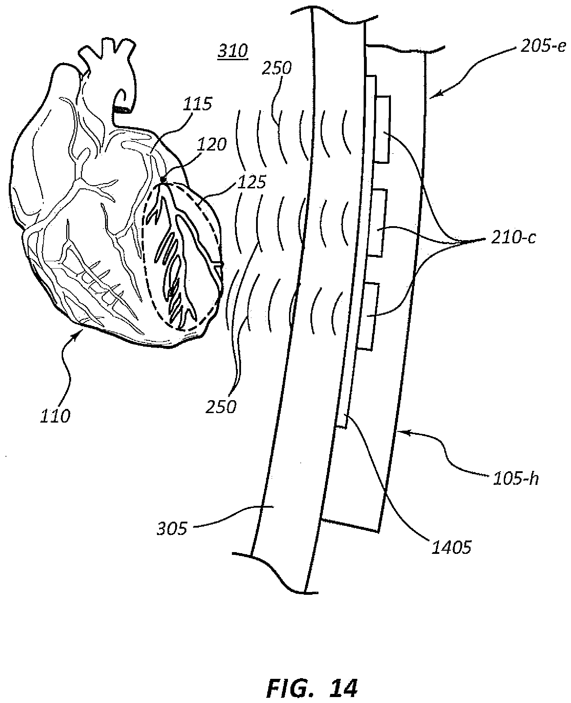

FIGS. 13 and 14 shows a heart treatment device 105-h, which may be an example of the heart treatment device 105 of FIG. 1. The heart treatment device 105-h includes a delivery member 205-e having a plurality of heating elements 210-c and a tissue interface portion 1405. The delivery member 205-e may be constructed as a vest or other article of clothing that the patient puts on to cover a portion of the upper body of the patient. The delivery member 205-e may position the plurality of heating elements 210-c in proximity to a typical location of the heart 110 within the patient. The heating elements 210-c may be individually or concurrently operable by the operator based on, for example, the size and shape of the patient relative to the size and shape of the delivery member 205-e. The heart treatment device 105-h may be connected to a power source 240. The power source 240 may be a battery or other mobile power source to provide the heart treatment device 105-h as a mobile device.

Any number of heating elements 210-c may be used. The embodiment of FIG. 13 includes 16 heating elements arranged in rows and columns. Other embodiments may include a different number of heating elements and any desired arrangement for the heating elements. In some embodiments, the heart treatment device 105-h may include only a single one of the heating element 210-c. The single one of the heating element 210-c may have an enlarged size as compared to some of the other embodiments disclosed herein with reference to the figures, wherein the enlarged size promotes heating of a larger portion of the heart 110 to ensure that the damaged heart tissue 125 is heated sufficiently to promote stimulation of the heart shock proteins.

The heating elements 210-c may be operated to direct energy waves 250 to the heart 110 as shown in FIG. 14. In at least some examples, the energy waves 250 generated by the heating elements 210-c may be less precise than some of the other heart treatment devices disclosed herein. However, the heating effect provided by the heart treatment device 105-h may still have measurable positive effects on the heart tissue 125. In one example, the heart treatment device 105-h may be used by emergency medical technicians in an ambulance while transporting a patient to the hospital for specific treatment of a heart attack. The heart treatment device 105-h may also be used in the military, such as during combat and field operations. Heating the heart 110 to promote stimulation of heat shock proteins as early as possible during and after a heart attack, such as during an ambulance ride, may result in improved salvaging of the myocardium, thereby reducing the infarct size. The heart treatment device 105-h may be used in other scenarios such as, for example, within an emergency room prior to and during a percutaneous catheter intervention procedure.

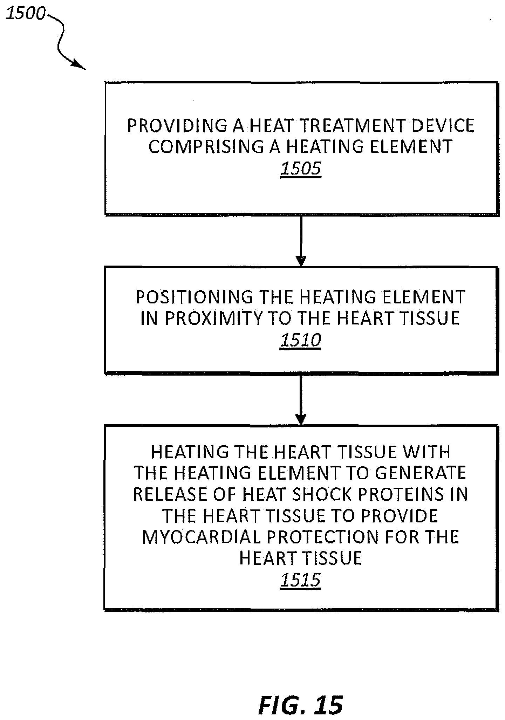

FIGS. 15-17 are flow diagrams showing example methods in accordance with the present disclosure. FIG. 15 is directed to a method 1500 of treating ischemia in heart tissue after a heart attack. The method includes a step 1505 of providing a heart treatment device comprising a heating element, and a step 1510 of positioning the heating element in proximity to the heart tissue. The method 1500 also includes a step 1515 of heating the heart tissue with the heating element to generate release of heat shock proteins in the heart tissue to provide myocardial protection for the heart tissue.

The method 1500 may also include heating the heart tissue by directing ultrasound waves into the heart tissue. The ultrasound waves may have a frequency in the range of about 1 MHz to about 10 MHz. The method may include delivering a drug to the heart tissue, wherein the drug comprises heat shock proteins. The method may include driving the drug into the heart tissue with the ultrasound waves. The method may include injecting the drug into the heart tissue (*e.g.,* using a needle). The drug may comprise an adenovirus vector.

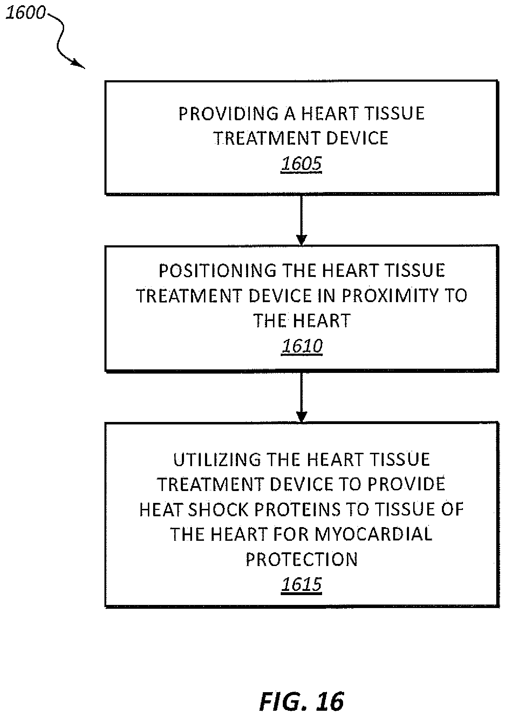

Referring to FIG. 16, another method 1600 is directed to providing myocardial protection in the heart after myocardial infarction. The method 1600 may include a step 1605 of providing a heart tissue treatment device, and a step 1610 of positioning the heart tissue treatment device in proximity to the heart. The method 1600 may include a step 1615 of utilizing the heart tissue treatment device to provide heat shock proteins to tissue of the heart for myocardial protection.

The method 1600 may also include positioning a portion of the heart tissue treatment device in contact with an exterior surface of the heart. The method 1600 may include heating the tissue using the heart tissue treatment device to generate the heat shock proteins. The heat shock proteins may include HSP70 protein. Positioning the heart tissue treatment device may include inserting a portion of the heart tissue treatment device through a vessel into the heart. Providing the heat shock proteins may include delivering the heat shock proteins via an adenoviral vector. The method 1600 may include providing a fluid interface between the heart tissue treatment device and a tissue of the heart.

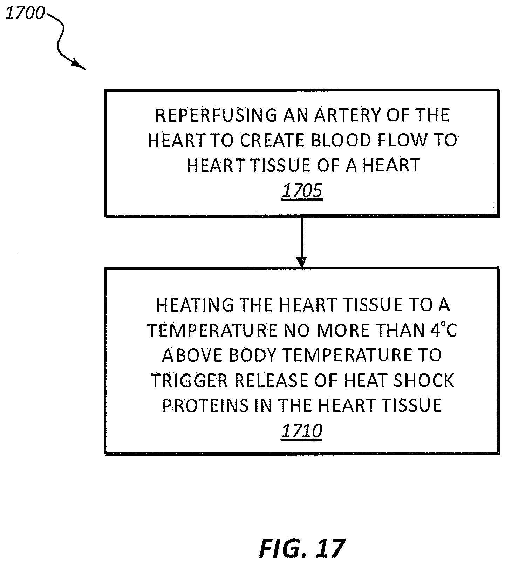

FIG. 17 is directed to another example method 1700 of treating heart tissue damage during a heart attack. The method 1700 may include a step 1705 of reperfusing an artery of the heart to create blood flow to the heart tissue. The method 1700 may also include a step 1710 of heating the heart tissue to a temperature no more than about 7°C above body temperature to trigger release of heat shock proteins in the heart tissue.

The method 1700 may include generating heat using one of an ultrasound device, a radio frequency device, a resisted heating device, a microwave device, and a laser heating member. Heating the heart tissue may comprise heating from outside of the heart. Heating the heart tissue may include heating from inside of the heart. The method 1700 may include heating the heart tissue to a temperature of about 37°C to about 42°C. Heating may be provided using a heating element, wherein the method further includes providing a fluid interface between the heating element and the heart tissue.

The method 1700 may also include providing a heating element and a fluid-filled balloon between the heating element and the heart tissue. The method 1700 may include providing a heating element carried by a catheter that is insertable through a vessel to the heart. The catheter may include a reperfusion member operable to provide the reperfusing. The heating element may be positioned on the catheter at a location distal of the reperfusion member.

Many other methods are possible for treating heart tissue using heat shock proteins. The heat shock proteins may be delivered to the heart tissue via an injected drug or by some other application. Alternatively, the heart tissue may be stimulated using, for example, heat wherein the heart tissue generates its own heat shock proteins. The heat shock proteins may provide myocardial protection for the heart tissue, which may be useful after a heart attack.

The detailed description set forth above in connection with the appended drawings describes example embodiments and does not represent the only embodiments that may be implemented or that are within the scope of the claims. The detailed description includes specific details for the purpose of providing an understanding of the described techniques. These techniques, however, may be practiced without these specific details. In some instances, well-known structures and devices are shown in block diagram form in order to avoid obscuring the concepts of the described embodiments.

The previous description of the disclosure is provided to enable a person skilled in the art to make or use the disclosure. Various modifications to the disclosure will be readily apparent to those skilled in the art, and the generic principles defined herein may be applied to other variations without departing from the spirit or scope of the disclosure. Throughout this disclosure the term "example" or "exemplary" indicates an example or instance and does not imply or require any preference for the noted example. Thus, the disclosure is not to be limited to the examples and designs described herein but to the scope of appended claim 1.

## Claims

1. A heat treatment system for treating heart tissue, comprising
a drug, wherein the drug comprises heat shock proteins or an adenovirus vector delivering a heat shock protein promoting gene; and
a heart treatment device (105-b,105-c,105-f) comprising an ultrasound device (210,210-a) for driving the drug into the heart and for generating, in use, heat in heart tissue for stimulating generation of heat shock proteins, the heart treatment device further comprising means for delivering the drug to the heart tissue.

2. A system as claimed in claim 1 wherein the ultrasound device (210,210-a) produces ultrasound waves having a frequency in the range of 1Mhz to 10MHz.

3. A system according to claim 1 or 2, wherein the heart treatment device (105-b,105-c,105-f) comprises a delivery member (205,205-c), the delivery member having a distal support portion on which the ultrasound device (210,210-a) is carried, and a tissue interface portion (215,215-a,705) configured to be positioned between the ultrasound device and the heart tissue in use.

4. A system according to claim 3, wherein the delivery member (205) comprises: a handle (230), an elongate support arm (225) extending from the handle and configured for insertion through a wall of a patient's chest cavity to access the heart, the distal support portion (220) being supported at a distal end of the elongate support arm.

5. A system according to claim 4, wherein the tissue interface portion (215) comprises a compliant structure for providing improved contact with an outer surface of the heart.

6. A system according to claim 5, wherein the tissue interface portion (215) comprises a balloon structure that is filled with fluid.

7. A system according to claim 4, 5 or 6, wherein the heart treatment device (105-b) further comprises a needle (405) for delivery of the drug directly to heart tissue.

8. A system according to claim 7, wherein the heart treatment device (105-b) further comprises a needle lumen (415), such that the needle (405) can be advanced from the needle lumen for delivery of the drug and thereafter retracted into the needle lumen.

9. A system according to claim 4, 5 or 6, wherein the tissue interface portion (215-a) comprises a fluid permeable material through which the drug can pass, the heart treatment device (105-c) further comprising a drug delivery lumen (505) for delivery of the drug to the tissue interface portion.

10. A system according to claim 3, wherein the delivery member (205-c) comprises a catheter configured for insertion through a vessel or other lumen into the heart.

11. A system according to claim 10, wherein the tissue interface portion (705) is an expandable balloon having a fluid path for delivery of inflation fluid into the tissue interface portion.

12. A system according to claim 11, wherein the heart treatment device (105-f) further comprises a pair of needles (905) for delivery of the drug to heart tissue.

13. A system according to claim 12, wherein the pair of needles (905) can be withdrawn back into the delivery member (205-c) after delivering the drug, or wherein the heart treatment device comprises separate needle lumens positioned outside of or carried by the delivery member within which the needles are carried.

14. A system according to any of claims 10 to 13, further comprising a reperfusion member (1005) positioned on the catheter proximal of the heating element.

15. A system according to claim 14, wherein the reperfusion member (1005) is a stent or an inflation balloon.

## Patentansprüche

1. Wärmebehandlungssystem zur Behandlung von Herzgewebe,
umfassend ein Arzneimittel, wobei das Arzneimittel Hitzeschockproteine oder einen Adenovirusvektor umfasst, der ein hitzeschockproteinförderndes Gen liefert; und
eine Herzbehandlungsvorrichtung (105-b, 105-c, 105-f), umfassend eine Ultraschallvorrichtung (210, 210-a) zum Lenken des Arzneimittels in das Herz und zum Erzeugen von Wärme im Herzgewebe, wenn in Gebrauch, um die Erzeugung von Hitzeschockproteinen zu stimulieren, wobei die Herzbehandlungsvorrichtung ferner Mittel zum Abgeben des Arzneimittels an das Herzgewebe umfasst.

2. System nach Anspruch 1, wobei die Ultraschallvorrichtung (210, 210-a) Ultraschallwellen mit einer Frequenz im Bereich von 1 MHz bis 10 MHz erzeugt.

3. System nach Anspruch 1 oder 2, wobei die Herzbehandlungsvorrichtung (105-b, 105-c, 105-f) ein Abgabeelement (205, 205-c) umfasst, wobei das Abgabeelement einen distalen Stützabschnitt, auf dem die Ultraschallvorrichtung (210, 210-a) getragen wird, und einen Gewebeschnittstellenabschnitt (215, 215-a, 705) aufweist, der konfiguriert ist, um in Gebrauch zwischen der Ultraschallvorrichtung und dem Herzgewebe positioniert zu werden.

4. System nach Anspruch 3, wobei das Abgabeelement (205) umfasst:
einen Griff (230), einen länglichen Stützarm (225), der sich vom Griff aus erstreckt und zum Einführen durch eine Wand der Brusthöhle eines Patienten konfiguriert ist, um Zugang zum Herzen zu erhalten, wobei der distale Stützabschnitt (220) an einem distalen Ende des länglichen Stützarms getragen wird.

5. System nach Anspruch 4, wobei der Gewebeschnittstellenabschnitt (215) eine fügsame Struktur zum Bereitstellen eines verbesserten Kontakts mit einer Außenfläche des Herzens umfasst.

6. System nach Anspruch 5, wobei der Gewebeschnittstellenabschnitt (215) eine Ballonstruktur umfasst, die mit Fluid gefüllt ist.

7. System nach Anspruch 4, 5 oder 6, wobei die Herzbehandlungsvorrichtung (105-b) ferner eine Nadel (405) zur Abgabe des Arzneimittels direkt an Herzgewebe umfasst.

8. System nach Anspruch 7, wobei die Herzbehandlungsvorrichtung (105-b) ferner ein Nadellumen (415) umfasst, so dass die Nadel (405) zur Abgabe des Arzneimittels aus dem Nadellumen vorgeschoben und danach in das Nadellumen zurückgezogen werden kann.

9. System nach Anspruch 4, 5 oder 6, wobei der Gewebeschnittstellenabschnitt (215-a) ein fluiddurchlässiges Material umfasst, durch das das Arzneimittel passieren kann, wobei die Herzbehandlungsvorrichtung (105-c) ferner ein Arzneimittelabgabelumen (505) zur Abgabe des Arzneimittels an den Gewebeschnittstellenabschnitt umfasst.

10. System nach Anspruch 3, wobei das Abgabeelement (205-c) einen Katheter umfasst, der zum Einführen durch ein Gefäß oder ein anderes Lumen in das Herz konfiguriert ist.

11. System nach Anspruch 10, wobei der Gewebeschnittstellenabschnitt (705) ein expandierbarer Ballon mit einem Fluidweg zur Abgabe von Inflationsfluid in den Gewebeschnittstellenabschnitt ist.

12. System nach Anspruch 11, wobei die Herzbehandlungsvorrichtung (105-f) ferner ein Paar von Nadeln (905) zur Abgabe des Arzneimittels an Herzgewebe umfasst.

13. System nach Anspruch 12, wobei das Paar von Nadeln (905) nach der Abgabe des Arzneimittels in das Abgabeelement (205-c) zurückgezogen werden kann oder wobei die Herzbehandlungsvorrichtung separate Nadellumen umfasst, die außerhalb des Abgabeelements, in dem die Nadeln getragen werden, positioniert sind oder von diesem getragen werden.

14. System nach einem der Ansprüche 10 bis 13, ferner umfassend ein Reperfusionselement (1005), das auf dem Katheter proximal des Heizelements positioniert ist.

15. System nach Anspruch 14, wobei das Reperfusionselement (1005) ein Stent oder ein Inflationsballon ist.

## Revendications

1. Système de traitement thermique pour traiter le tissu cardiaque,
comprenant un médicament, le médicament comprenant des protéines de choc thermique ou un vecteur adénovirus administrant un gène favorisant une protéine de choc thermique ; et
un dispositif de traitement cardiaque (105-b, 105-c, 105-f) comprenant un dispositif à ultrasons (210 210-a) pour entraîner le médicament dans le cœur et pour générer, en cours d'utilisation, de la chaleur dans le tissu cardiaque pour stimuler la génération de protéines de choc thermique, le dispositif de traitement cardiaque comprenant en outre un moyen pour administrer le médicament au tissu cardiaque.

2. Système selon la revendication 1, dans lequel le dispositif à ultrasons (210, 210-a) produit des ondes ultrasonores ayant une fréquence dans la plage de 1 MHz à 10 MHz.

3. Système selon la revendication 1 ou 2, dans lequel le dispositif de traitement cardiaque (105-b, 105-c, 105-f) comprend un organe d'administration (205, 205-c), l'organe d'administration ayant une partie de support distale sur laquelle le dispositif à ultrasons (210, 210-a) est porté, et une partie d'interface tissulaire (215, 215-a, 705) est conçue pour être positionnée entre le dispositif à ultrasons et le tissu cardiaque en cours d'utilisation.

4. Système selon la revendication 3, dans lequel l'organe d'administration (205) comprend :
une poignée (230), un bras de support allongé (225) s'étendant depuis la poignée et conçu pour être inséré à travers une paroi de la cavité thoracique d'un patient pour accéder au cœur, la partie de support distale (220) étant soutenue à une extrémité distale du bras de support allongé.

5. Système selon la revendication 4, dans lequel la partie d'interface tissulaire (215) comprend une structure souple pour fournir un contact amélioré avec une surface externe du cœur.

6. Système selon la revendication 5, dans lequel la partie d'interface tissulaire (215) comprend une structure de ballonnet qui est remplie de fluide.

7. Système selon la revendication 4, 5 ou 6, dans lequel le dispositif de traitement cardiaque (105-b) comprend en outre une aiguille (405) pour l'administration du médicament directement au tissu cardiaque.

8. Système selon la revendication 7, dans lequel le dispositif de traitement cardiaque (105-b) comprend en outre une lumière d'aiguille (415), de telle sorte que l'aiguille (405) peut être avancée depuis la lumière d'aiguille pour l'administration du médicament et ensuite rétractée dans la lumière d'aiguille.

9. Système selon la revendication 4, 5 ou 6, dans lequel la partie d'interface tissulaire (215- a) comprend un matériau perméable aux fluides à travers lequel le médicament peut passer, le dispositif de traitement cardiaque (105-c) comprenant en outre une lumière d'administration de médicament (505) pour l'administration du médicament à la partie d'interface tissulaire.

10. Système selon la revendication 3, dans lequel l'organe d'administration (205-c) comprend un cathéter conçu pour être inséré à travers un vaisseau ou une autre lumière dans le cœur.

11. Système selon la revendication 10, dans lequel la partie d'interface tissulaire (705) est un ballonnet dilatable ayant un trajet de fluide pour l'administration de fluide de gonflage dans la partie d'interface tissulaire.

12. Système selon la revendication 11, dans lequel le dispositif de traitement cardiaque (105-f) comprend en outre une paire d'aiguilles (905) pour l'administration du médicament au tissu cardiaque.

13. Système selon la revendication 12, dans lequel la paire d'aiguilles (905) peut être retirée dans l'organe d'administration (205-c) après l'administration du médicament, ou dans lequel le dispositif de traitement cardiaque comprend des lumières d'aiguille séparées positionnées à l'extérieur ou portées par l'organe d'administration à l'intérieur duquel les aiguilles sont portées.

14. Système selon l'une quelconque des revendications 10 à 13, comprenant en outre un organe de reperfusion (1005) positionné sur le cathéter à proximité de l'élément chauffant.

15. Système selon la revendication 14, dans lequel l'organe de reperfusion (1005) est une endoprothèse ou un ballonnet de gonflage.
